Europäisches Patentamt

European Patent Office

Office européen des brevets

(19)

(11) Publication number: **0 005 030**
**B1**

(12) **EUROPEAN PATENT SPECIFICATION**

(45) Date of publication of patent specification: **18.01.84**

(21) Application number: **79300602.4**

(22) Date of filing: **11.04.79**

(51) Int. Cl.³: **C 12 P 19/06,**
**C 08 B 37/00, A 61 K 7/16,**
**A 23 L 1/04**

(54) Low calcium xanthan gums.

(30) Priority: **13.04.78 US 895907**

(43) Date of publication of application:
**31.10.79 Bulletin 79/22**

(45) Publication of the grant of the patent:
**18.01.84 Bulletin 84/3**

(84) Designated Contracting States:
**BE CH DE FR GB IT LU NL SE**

(56) References cited:
**FR - A - 2 360 665**

(73) Proprietor: **MERCK & CO. INC.**
**126, East Lincoln Avenue P.O. Box 2000**
**Rahway New Jersey 07065 (US)**

(72) Inventor: **Richmon, Joe B.**
**448 Sandlewood Drive**
**El Cajon California 92021 (US)**

(74) Representative: **Crampton, Keith John Allen et al,**
**D YOUNG & CO 10 Staple Inn**
**London WC1V 7RD (GB)**

Courier Press, Leamington Spa, England.

## Low calcium xanthan gums

This invention relates to xanthan gum. The preparation and uses of xanthan gum are well known to those skilled in the field of heteropolysaccharides. While aqueous compositions of xanthan gum have many desirable properties, such compositions have a chunky or non-uniform flow.

The present invention is based on the discovery that there is a correlation between the calcium content of xanthan gum and the flow characteristics of aqueous compositions containing xanthan gum.

The xanthan gum of the present invention has not more than 400 ppm (0.04 weight per cent) and not less than 37 ppm (0.0037 weight per cent) of calcium. In the gum of the invention, not more than 1.6% of the xanthan gum carboxyl groups are bound to calcium ions. The gum of the invention is prepared from an aqueous fermentation medium substantially but not completely free of calcium ion and substantially free of calcium-containing fermentation nutrients which contain calcium. When prepared under conditions of high shear the gum provides aqueous oil/water compositions having smooth or uniform flow.

In the specification the term "ppm" means "parts per million by weight", mesh sizes are U.S. standards, and all parts and percentages are by weight.

Aqueous compositions of xanthan gum tend to have a "chunky" or non-uniform flow characteristic. A "chunky" flow is an uneven, lumpy type of flow such as is encountered with tomato ketchup. A smooth or uniform type of flow is one free of lumps and unevenness such as is encountered with vegetable oil.

The aqueous compositions include solutions of xanthan gum as well as oil/water emulsions. In general, aqueous compositions of xanthan gum containing not more than 0.04 weight % of calcium and fermented under high shear conditions have desirable flow properties, and aqueous compositions of xanthan gum containing not more than 0.02 weight % of calcium and fermented under high shear conditions have best flow properties.

The low calcium xanthan gum of the present invention may be prepared by a heteropolysaccharide-producing bacterium of the genus *Xanthomonas* by the whole culture fermentation of a medium comprising a fermentable carbohydrate, a nitrogen source, and appropriate other nutrients. The bacterium may be any suitable *Xanthomonas* species preferably *X. campestris.*

The bacterium is grown in a medium which is substantially (but not completely) free of calcium ions. By substantially free is meant up to 4 ppm of calcium ion per each 1% of xanthan gum concentration in the completed fermentation broth, and preferably up to 2 ppm of calcium per each 1% of xanthan gum concentration in the completed fermentation broth. Thus, if the xanthan gum is to be produced at a final concentration of 2.1—2.3%, the total calcium ion content of the completed fermentation broth should not exceed 9 ppm and preferably should not exceed 5 ppm. To obtain such a low calcium medium the calcium content of the water in the fermentation medium may be reduced to the appropriate level by chemical means, e.g. ion-exchange treatment, or by distillation. As commercial sources of organic nitrogen contain appreciable amounts of calcium ion, it is important that the nitrogen source of the present invention be a material which is substantially free of calcium ions. An example of such a nutrient material is Promosoy 100, a soy protein concentrate (Central Soya). Use of this material at 500 ppm imparts 1—2 ppm calcium to the medium.

The relationship between the total calcium ion content of the fermentation medium, the final xanthan gum concentration in the broth, and the calcium ion content of the isolated xanthan gum is expressed in Table 1.

TABLE 1

Calcium ion relationships

| Total calcium ion of medium (ppm) | Final xanthan gum concentration (%) | Calcium content of xanthan gum (ppm) |
|---|---|---|
| 12 | 3 | 400 |
| 8 | 2 | 400 |
| 4 | 1 | 400 |
| 6 | 3 | 200 |
| 4 | 2 | 200 |
| 2 | 1 | 200 |

Prior-art fermentations of xanthan gum failed to appreciate the benefits obtainable by low concentrations of calcium and, indeed, teach the addition of calcium either to the fermentation beer or to the reconstituted xanthan gum. Examples of such prior-art teachings are U.S.—A—3,000,790, 3,054,689, 3,096,293, 3,232,929 and 4,053,699 and FR—A—2,330,697.

In addition the prior art teaches the use of tap water rather than deionized water, not only because of economic considerations, but because tap water contains trace elements required for growth of the gum-producing organism. See, for example, "Polysaccharide (Xanthan) of *Xanthomonas campestris* NRRL B-1459: Procedures for Culture Maintenance and Polysaccharide Production, Purification and Analysis", Agricultural Research Service, United States Department of Agriculture (ARS-NC-51).

Moreover, the prior art teaches the use of distillers' solubles or soybean cake as an organic nitrogen source for the fermentation of xanthan gum. See, for example, U.S.—A—3,020,206, 3,281,329 and 3,594,280 and "Materials and Methods in Fermentation", pp. 126—127 by G. L. Solomons, Academic Press, New York (1969). At a concentration in the fermentation broth of 0.4 weight %, Distillers Dried Solubles imparts a calcium content of 150.8 ppm to the fermentation broth while at a concentration of 0.45 weight % in the fermentation broth soybean meal imparts a calcium content of 16.7 ppm to the fermentation broth. The gum produced at a concentration of 2.1—2.3% with the use of such organic nitrogen sources would have a calcium content of 0.66—0.72% calcium in the case of Distillers Dried Solubles and 0.07—0.08% in the case of soybean meal because xanthan gum binds all available calcium ion up to a maximum level of 2.6%, assuming that no other calcium is present in the water or other components of the medium.

Xanthan gum is an anionic polysaccharide due to the presence of 20% glucuronic acid and 4% pyruvate in the molecule. It has been experimentally determined that 0.026 g calcium will react with all of the carboxyl groups in 1 g of xanthan gum. In other words this amount of calcium is the stoichiometric amount based on the carboxyl groups in the xanthan gum molecule. From this relationship it can be calculated that for each 1% of xanthan gum in the final fermentation broth, a calcium concentration in the broth of 260 ppm is the stoichiometric quantity sufficient to react with all of the carboxyl groups in the xanthan gum molecule. The gum recovered from such a broth will have a calcium content of 26,000 ppm. The % of carboxyl groups that will react with diminishing amounts of calcium can likewise be calculated. The relationship of calcium content to % of carboxyl groups bound is shown in Table 2.

TABLE 2
$Ca^{++}$ Concentration v. % carboxyl bound

| Total Ca content of media (ppm) | Xanthan gum concentration (%) | Ca content of Xan. gum (ppm) | % Carboxyl groups bound |
|---|---|---|---|
| 260 | 1 | 26,000 | 100 |
| 22 | 1 | 2,200 | 8.5 |
| 7 | 1 | 700 | 2.7 |
| 4 | 1 | 400 | 1.6 |
| 2 | 1 | 200 | 0.8 |
| 520 | 2 | 26,000 | 100 |
| 44 | 2 | 2,200 | 8.5 |
| 14 | 2 | 700 | 2.7 |
| 8 | 2 | 400 | 1.6 |
| 4 | 2 | 200 | 0.8 |
| 650 | 2.5 | 26,000 | 100 |
| 55 | 2.5 | 2,200 | 8.5 |
| 17.5 | 2.5 | 700 | 2.7 |
| 10 | 2.5 | 400 | 1.6 |
| 5 | 2.5 | 200 | 0.8 |

Thus, the xanthan gum of the present invention can be described chemically as xanthan gum in which up to 1.6% of the carboxyl groups are bound to calcium and the remaining carboxyl groups are bound to sodium, potassium, a mixture of sodium and potassium or other non-calcium cations.

The smooth flow obtainable with the low calcium xanthan gum of the present invention is liable, in some cases, to be degraded by high temperature pasteurization conditions. For this reason, it is preferable to pasteurize at temperatures which do not exceed 80°C.

During the fermentation of xanthan gum, the fermentation broth is continually monitored to assure good mixing. As the viscosity of the broth increases with the amount of gum produced, frequent monitoring and a corresponding increase in agitation rate assures that all parts of the broth are properly aerated. The criterion of good mixing, well known to those skilled in the polysaccharide fermentation art, is sufficient to produce the low calcium xanthan gum of this invention.

When it is desired to produce the low calcium xanthan gum having smooth flow properties, high shear is required during the fermentation process. The following agitation conditions have been found to be adequate to produce the low calcium, smooth flow xanthan gum of this invention. Agitation comparable to these agitation conditions is defined herein as "high shear".

| Fermentor size | Agitation conditions |
| --- | --- |

3.8 litres — Three 8-cm flat turbine impellors. The initial agitation is set at 400 RPM (32 metres/min) and is typically increased to 800—1000 RPM (64 to 80 metres/min) by 16—24 hours.

14 litres — Three 7.5-cm flat-blade impellors. The fermentation is started with an agitation rate of 400 RPM (30 metres/min) and is typically increased to 1000 RPM (75 metres/min) by 16—24 hours. The agitation can be increased as necessary to provide high shear up to 1500 RPM (112 metres/min).

30 litres — Two 12.85-cm V-shaped turbine impellors. The initial agitation is 300 RPM (37.5 metres/min) which is increased to 700 RPM (87.5 metres/min) by 16—24 hours.

70 litres — Two 15-cm flat blade turbine impellors and one 15.25-cm propellor. This fermentor is started with an agitation rate of 300 RPM (45 metres/min) and increased to 600 RPM (90 metres/min) by 16—24 hours. It can be increased thereafter as needed to provide high shear to a maximum of 750 RPM (122.5 metres/min).

The high shear must be imparted to the beer during the fermentation process. If the beer is subjected to high shear after the fermentation is completed, the resulting gum does not exhibit smooth flow. Likewise, it is preferred to continue the high-shear conditions throughout the entire fermentation process.

A correlation has been found between the smooth flow property of the xanthan gum of this invention and the viscosity of an oil/water emulsion made up from the gum. The following test protocol can therefore be followed to determine whether a low calcium xanthan gum can also be characterized as having smooth flow.

Test method 1

3.5 g of low calcium xanthan gum is slurried in 20 g of vegetable oil. The slurry is added to 300 ml tap water in a Sunbeam solid state Waring blender and mixed for 20 seconds at the lowest speed (stir button). Mixing is stopped, 13 g of NaCl is added, and the mix is agitated at the highest speed (liquefy button) for 10 seconds. The entire emulsion is poured into a 400 ml beaker and viscosity readings are obtained at room temperature on a Brookfield LVF viscometer, spindle 3 at 60 rpm. The xanthan gum used should contain between 86 and 92% solids and should be milled so that at least 98% passes through an 80 mesh screen and less than 40% passes through a 325 mesh screen. A low calcium xanthan gum is smooth flow if under these conditions viscosity readings of less than 1650 mPa.s (cP) are obtained. It is preferred that the viscosity be less than 1600 mPa.s (cP).

Alternative, although less reliable, tests require visual observations. For example, the emulsion prepared as above is observed while being poured and its flow characteristics noted. A beaker containing such an emulsion is swirled so that its sides are coated and then the sides are observed. If the coating of emulsion on the sides is generally homogenous rather than streaked and uneven, the gum can be considered to be smooth flow.

The low-calcium xanthan gum of this invention can be used for any of the uses to which xanthan gum can be put. In addition, when still in the fermentation broth it is the intermediary for producing the smooth-flow low calcium xanthan gum of this invention.

Smooth-flow xanthan gum finds applicability in a variety of areas. First, to the extent that its properties are similar to those of xanthan gum, it can be used as a substitute in formulations requiring xanthan gum. However, the smooth-flow xanthan gum of this invention is particularly useful in pourable and spoonable salad dressings. Solubility is markedly improved in reconstituted dry mixes such as fruit-flavored beverages, cocoa drinks, gravies, and soups. Texture and flow properties are markedly improved in high-sugar/solids systems such as sugar syrups, toothpaste, shampoo, hand cream, and fruit preserves. Representative usage levels are:

|  | % by weight |
| --- | --- |
| No/low oil sald dressing | 0.5—1.5 |
| High oil salad dressing | 0.2—0.8 |
| Toothpaste | 0.7—2.0 |
| preferably | 1.0—1.2 |
| Dry Foodstuffs (dispersible) | 0.2—1.5 |

Representative formulations using smooth-flow xanthan gum of this invention are as follows:

Salad dressing

To 40 parts of sugar add 20 parts of instant starch and 5 parts of smooth-flow xanthan gum. Dry blend and then disperse in 410 parts of water. Mix until dissolved and add 60 parts of sugar. Mix in 20 parts of salt, 5 parts of mustard and 40 parts of fresh egg yolks. Using a fast whip beater mix in 300 parts of corn oil and 100 parts of 100 grain vinegar.

Orange flavored drink mix

A drink is prepared by adding the following blended ingredients to 1 quart (944 ml) cold water and stirring for 30 seconds.

|  | Gms. |
|---|---|
| Baker's Special Sugar | 125.713 |
| Citric Acid, granulated anhydrous | 4.55 |
| Sodium Citrate, fine granular hydrous | 1.05 |
| Artificial Orange Juice Flavor 24825 (American Flavor and Fragrance Corp.) | 0.504 |
| Ascorbic Acid | 0.49 |
| Orange Essence Oil 1939 (Borden) | 0.336 |
| Smooth-Flow Xanthan Gum | 0.28 |
| Kowet Titanium Dioxide (Kohnstamm) | 0.042 |
| FD&C Yellow No. 5 | 0.021 |
| FD&C Yellow No. 6 | 0.014 |
|  | 133.0 |

Toothpaste

Using known processes, a toothpaste is prepared from the following ingredients:

|  | % by weight |
|---|---|
| Dicalcium phosphate dihydrate | 45.0 |
| Glycerine | 12.5 |
| Sorbitol | 12.5 |
| Sodium lauryl sulfate | 1.5 |
| Saccharin | 0.2 |
| Flavoring agent | 1.0 |
| Water | 26.3 |
| Smooth-Flow Xanthan Gum | 1.0 |

The resulting paste has short, non-stringy flow, whereas a paste made with regular xanthan gum has noticeable stringiness.

Instant hot cream soup mix

An instant soup is prepared by adding the following blended ingredients to 3/4 cup (180 ml) boiling water and stirring for 2 minutes.

|  | Gms. |
|---|---|
| Star Dri 24F Corn Syrup Solids (Staley) | 6.56 |
| Veg Cream (types) (Nestle) | 3.75 |
| Gelatinized Dura Jel (Staley) | 3.00 |
| Milk Solids Non-fat (instantized) | 2.50 |
| Salt | 1.50 |
| Sugar | 1.00 |
| MSG | 0.90 |
| Powdered onion | 0.20 |
| Maggi Hydrolyzed Plant Protein (Nestle) | 0.10 |
| Smooth flow xanthan gum | 0.25 |

The following examples further illustrate the present invention without, however, limiting the same thereto.

Example 1

All the media and media ingredients are prepared using the following procedure in deionized water to minimize the presence of calcium.

The flask seed medium is YM broth (Difco). These flasks are inoculated with a loopful of a strain of *Xanthomonas campestris* NRRL B-1459 grown on nutrient agar (Difco) or YM agar plates. The inoculated flasks are placed on a gyrotary shaker (New Brunswick Scientific, Inc.) at a shaking speed of 200—300 rpm. The temperature is controlled at 28—33°C.

After 18—40 hours these seeds are used to inoculate a five-liter fermentor vessel containing three liters of a medium containing the following ingredients:

| | |
|---|---|
| 3.0% | Dextrose |
| 0.05% | Promosoy 100 (Central Soya) |
| 0.09% | $NH_4NO_3$ |
| 0.5% | $Na_2HPO_4$ |
| 0.01% | $MgSO_4 \cdot 7H_2O$ |

The medium contains trace elements such as $BO_3^{\equiv}$, $Mn^{++}$, $FE^{++}$, $Cu^{++}$, $Zn^{++}$, $Co^{++}$, $MoO_4^{=}$, and an antifoam agent (Sag 471).

The fermentation temperature is controlled at 28—33°C with the agitation rate set so that proper mixing of the fermentor contents occurs. Sterile air is supplied at a rate of 0.2—1.0 (v/v). After 18—40 hours, this seed is used to inoculate either 50L of similar medium in a 70L fermentor, or 20L of such medium in a 30L fermentor. This medium is composed of the following ingredients:

| | |
|---|---|
| 3.0% | Dextrose |
| 0.5% | $Na_2HPO_4$ |
| 0.9% | $NH_4NO_3$ |
| 0.01% | $MgSO_4 \cdot 7H_2O$ |
| 0.05% | Promosoy 100 |

In these fermentors, the pH is controlled in the range of 6.0—7.5 using KOH addition. Aeration is similar to that in the smaller fermentor. The agitation is increased as necessary to maintain high shear. The agitation in the 30L and 70L fermentors is as defined above for "high shear". The fermentation is terminated when the carbon source has been fully utilized.

The fermentation broth is pasteurized in the fermentation vessel at 75°C for 15 minutes. The product is recovered by alcohol precipitation. The recovered fibers are dried for 2 hours in a steam oven at 55°C followed by milling through a 20-mesh screen. This product, designated sample 1, is a low-calcium smooth-flow xanthan gum.

A second batch is prepared following the procedure of sample 1 but using Distillers Solubles as the organic nitrogen source and tap water instead of deionized water. This product, sample 2, is representative of commercially available xanthan gum.

A third batch is prepared using the same conditions as those used to prepare sample 1 except that deionized water containing 40 ppm added calcium is used in the media. This product, designated sample 3, is also comparable to commercially available xanthan gum.

Example 2
1. Seed Preparation

Fresh YM agar plate cultures of *X. campestris* B-1459 are used to inoculate YM broth flasks. The inoculated flasks are placed on a gyrotary shaker at a shaking speed of 200—300 rpm. At 24—30 hrs., these flasks are used to inoculate flasks containing the following components:

| | |
|---|---|
| Starch | 2.67% |
| $Na_2HPO_4$ | 0.50% |
| Promosoy 100 | 0.19% |
| $NH_4NO_3$ | 0.09% |
| NZ Amine A | 0.03% |
| $MgSO_4 \cdot 7H_2O$ | 0.02% |
| $FeSO_4 \cdot 7H_2O$ | 5 ppm |
| HoLe salts | 1 ml/L |
| Balab | 0.26% |
| Defoamer (Sag) | 0.02% |
| *Tap Water | ≈96.22% |

*The starch slurry is prepared in tap water for hydrolysis and represents 10% of the final fermentor volume.

The fermentation temperature is controlled at 28—33°C with the agitation rate set so that proper mixing of the fermentor contents occurs. Sterile air is supplied at a rate of 0.2—1.0 (v/v). The flasks are used at 24—35 hrs. to inoculate 14-liter fermentors with a 6—7% inoculum level.

2. Final fermentor

Fermentors of a 14-liter capacity are used for the final fermentation containing about 10 liters of the following medium:

| | |
|---|---|
| Corn syrup | 4.2% |
| $Na_2HPO_4$ | 0.053% |
| Promosoy 100 | 0.0336% |
| $MgSO_4 \cdot 7H_2O$ | 0.02% |
| $NH_4NO_3$ | 0.106% |
| $FeSO_4 \cdot 7H_2O$ | 5 ppm |
| HoLe salts | 1 ml/L |
| Defoamer (Sag) | 0.01% |
| Deionized water | ≈95.58% |

The $FeSO_4 \cdot 7H_2O$ and HoLe salts are autoclaved separately. Alternatively, they are filtered instead of being added directly to the medium to be autoclaved.

The pH is controlled with 25% NaOH or KOH at 6.0—7.5. Aeration is similar to that in the smaller fermentor.

Agitation is as defined above for "high shear" for a 14-litre fermentor.

Fermentation is terminated when the carbon source is less than 0.1%.

The fermentation broth is pasteurized in the fermentation vessel at 75°C for 15 minutes. The product is recovered by alcohol precipitation. The recovered fibers are dried for 2 hours in a stream oven at 55°C followed by milling through a 20-mesh screen. The product is a low-calcium smooth-flow xanthan gum.

Example 3

Xanthan gum samples are prepared under varying calcium levels.

The viscosities of the gum samples (1% and 2% w/w, in deionized (D.I.) water and 1% w/w KCl solution) and low oil emulsions are determined using a Brookfield LVF viscometer, at 60 rpm and appropriate spindle.

A slurry of each gum (6.4 g) in Kraft vegetable oil-specially processed soybean oil (40 g) is added to 500 ml of water with stirring. After hydration, sodium chloride (26 g) and 10% acetic acid (75 ml) are added. The emulsions are milled in a Sterling colloid mill at a setting of 0.015" (0.381 mm).

Working yield values are determined from the viscosity profiles at low shear rates obtained by using the spring relaxation method on the Wells-Brookfield RVT plate and cone viscometer. (See Jeanes et al., (1973) J. Appl. Polymer Sci. *17* pp. 1621—1624. The working yield value is defined as the shear stress (Pa) required to produce a shear rate of $0.01 \ sec^{-1}$.

Visual observation of flow properties are determined by pouring low oil emulsions from container to container. Flow properties are rated as "smooth" through "light chunky", "medium chunky" to "heavy chunky".

A quantitative determination of flow properties is carried out by measurement of the flow rate of gum solutions (1% w/w in 1% KCl) and emulsions in a Bostwick Consistometer. This instrument which is available from Central Scientific Co., Inc., 26005 Kostner Avenue, Chicago, Illinois 60623 determines consistency by measuring the distance that a material flows under its own weight during a given time interval. The distance travelled by the moving front after 5 minutes is a reproducible measure of the flow properties (degree of smoothness) of the solutions.

|  | Sample 4 | Sample 5 | Sample 6 |
|---|---|---|---|
| Calcium Content (ppm) | 130 | 2322 | 43572 |
| Magnesium Content (ppm) | 391 | 914 | 1019 |
| 1% Viscosity, D.I. $H_2O$ [mPa.s (cP), 60 rpm, spindle 3] | 410 | 810 | 815 |
| 1% Viscosity, 1% KCl [MPa.s (cP), 60 rpm, spindle 3] | 1075 | 1250—1550 | 1175 |
| Working Yield Value, 1% Gum, D.I. $H_2O$ (Pa) | (1) | 1.55 | 2.6 |
| Working Yield Value, 1% Gum, 1% KCl (Pa) | 2.2 | 5.2 | 4.2 |
| 2% Viscosity, D.I. $H_2O$ [mPa.s (cP), 60 rpm] | 1040 | N.D. | 1710 |
| 2% Viscosity, 1% KCl (cP), 60 rpm, spin 4.] | 4070 | N.D. | 4080 |
| 2% D.I. Solution Visual Flow Characteristic | Smooth | Chunky | Chunky |
| Working Yield Value, 2% Gum, D.I. $H_2O$ (Pa) | 1.6 | N.D. | 5.2 |

(1) Too low to determine due to low viscosity [<800 mPa.s (cP)] in D.I. water
N.D.=Not Determined

|  | Sample 4 | Sample 5 | Sample 6 |
|---|---|---|---|
| Visual Flow Characteristic of Low Oil Emulsion | Smooth | Medium to Heavy Chunky | Light to Medium Chunky |
| Low Oil Emulsion Viscosity (60 rpm) | 1390 | 1410 | 1490 |
| Low Oil Emulsion Working Yield Value (Pa) | 4.5 | N.D. | 8.2 |
| Bostwick Test, Distance Covered After 5 min (cm) |  |  |  |
| 1% Gum and 1% KCl | 17.2 | 10.9 | 12.7 |
| Low Oil Emulsion | 13.2 | N.D. | 9.7 |

N.D.=Not Determined

Example 4

A series of nine fermentations is carried out using deionized water for Run 1, and deionized water together with increasing ratios of tap water for Runs 2—9. The proteinaceous nutrient is Promosoy 100. At the end of the fermentation the gum in each sample is recovered, analyzed for calcium content, and its flow property determined visually using the procedure of Example 1. As the Promosoy 100 is substantially devoid of calcium, essentially all of the calcium found upon analysis is derived from the tap water. The following results are obtained:

| Run | Ca (ppm) | Flow properties (low oil/$H_2O$ emulsion) |
|---|---|---|
| 1 | 37 | Smooth |
| 2 | 123 | Slightly Chunky |
| 3 | 126 | Smooth |
| 4 | 193 | Slightly Chunky |
| 5 | 207 | Smooth |
| 6 | 315 | Slightly Chunky |
| 7 | 315 | Slightly Chunky |
| 8 | 478 | Chunky |
| 9 | 536 | Chunky |

Example 5

X. campestris is fermented under the low-calcium high-shear conditions of this invention. Following fermentation, samples of the fermentation broth are removed from the fermentor and

# 0 005 030

pasteurized at various temperatures and times using the copper coil immersed in a hot oil bath. The fermentation broth remaining in the fermentor is pasteurized in place at 75°C for various times. Using these procedures, the following samples are prepared:

| | |
|---|---|
| BD-118 | No pasteurization (control) |
| BD-122 | 75°C for 2—3 minutes in coil |
| BD-123 | 75°C for 10 minutes in coil |
| BD-124 | 79°C for 2—3 minutes in coil |
| BD-125 | 99°C for 2—3 minutes in coil |
| BD-119 | 75°C for 2—3 minutes in fermentor |
| BD-120 | 75°C for 5 minutes in fermentor |
| BD-121 | 75°C for 15 minutes in fermentor |

The xanthan gum in these samples is precipitated with alcohol. The fibrous product is dried overnight in a 45°C oven followed by milling through a 20-mesh screen.

| Sample No. | Calcium content (ppm) | Pasteurization conditions | Emulsion viscosity mPa.s (cP) | Emulsion flow properties | |
|---|---|---|---|---|---|
| | | | | Visual | Bostwick |
| BD-118 | 145 | No pasteurization | 1165 | Smooth | 14.0 |
| BD-122 | 145 | 75°C, 2—3 min., coil | 1215 | Smooth | 13.8 |
| BD-123 | 140 | 75°C, 10 min., coil | 1340 | Smooth | 12.1 |
| BD-124 | 140 | 79°C, 2—3 min., coil | 1240 | Smooth | 12.5 |
| BD-125 | N.D. | 99°C, 2—3 min, coil | 1215 | Smooth | 12.8 |
| BD-119 | 135 | 75°C, 2—3 min., steam | 1310 | Smooth | 12.7 |
| BD-120 | N.D. | 75°C, 5 min., steam | 1180 | Smooth | 13.3 |
| BD-121 | 140 | 75°C, 15 min., steam | 1270 | Smooth | 13.6 |

N.D. Not determined

Note: These samples were prepared from the same batch of broth, and therefore should have identical Ca contents. This is confirmed by analysis for calcium, which results are indicated above, wherein differences are within experimental error.

Example 6

*X. campestris* is fermented under the low calcium high shear conditions of this invention. Following fermentation, samples of the fermentation broth are removed from the fermentor and pasteurized at various times and temperatures by passing the broth through a copper coil immersed in a hot oil bath, followed by rapid cooling using an ice bath. The following samples are prepared:

| | |
|---|---|
| BD-107 | 79°C for 2—3 minutes |
| BD-108 | 85°C for 2—3 minutes |
| BD-109 | 91°C for 2—3 minutes |
| BD-110 | 99°C for 2—3 minutes |
| BD-111 | 79°C for 10 minutes |
| BD-112 | 116°C for 2—3 minutes |
| BD-113 | 99°C for 4—5 minutes |
| BD-115 | No pasteurization (control) |

The xanthan gum in these samples is precipitated with alcohol, dried for two hours in a steam oven at 55°C, and milled through a 20-mesh screen.

| Sample No. | Emulsion viscosity mPa.s (cP) | Emulsion flow properties | |
|---|---|---|---|
| | | Visual | Bostwick |
| BD-107 | 1610 | Light-medium | 10.5 |
| BD-108 | 1615 | Light-medium | 10.8 |
| BD-109 | 1535 | Light-medium | 10.5 |
| BD-110 | 1735 | Light-medium | 9.85 |
| BD-111 | 1540 | Light | 10.75 |
| BD-112 | 1635 | Light medium | 10.3 |
| BD-113 | 1600 | Medium | 10.6 |
| BD-115 | 1360 | Smooth | 13.6 |

These samples are prepared from the same broth and therefore have identical Ca content.

Example 7

In order to demonstrate that the smooth flow property is dependent upon calcium ion content of the fermentation broth in which the xanthan gum is produced, and is independent of Mg ion content, the following comparisons are made with emulsions made using xanthan gum prepared from a broth having the indicated level of Ca and Mg.

| Sample | $Ca^{++}$ | $Mg^{++}$ | Ca/Mg ratio | Flow properties of low oil emulsion |
|---|---|---|---|---|
| 7 | 4347 | 1019 | 4.28 | Medium-Heavy Chunky |
| 8 | 2322 | 914 | 2.54 | Medium-Heavy Chunky |
| 9 | 1866 | 917 | 2.03 | Medium-Heavy Chunky |
| 10 | 772 | 388 | 1.99 | Light Chunky |
| 11 | 567 | 546 | 1.04 | Medium Chunky |
| 12 | 261 | 594 | 0.44 | Smooth |
| 13 | 153 | 546 | 0.28 | Smooth |
| 14 | 130 | 391 | 0.33 | Smooth |

Samples which have smooth flow properties are characterized by $Ca^{++}$ levels of 261 ppm or below.

Example 8

An oil-well drilling fluid is made up in conventional manner from the following constituents: low calcium, smooth flow xanthan gum, 0.34 kg; water, 189.27 liters; betonite, 3.63 kg; carboxymethyl cellulose 0.23 kg; and KCl, 4.76 kg. The mud exhibits the following properties:

| Fann viscometer dial readings | Initial | Rolled 16 hrs. at 65.5°C (150°F) |
|---|---|---|
| 600 rpm | 31.3 | 27.7 |
| 300 rpm | 22.7 | 20.7 |
| 200 rpm | 19.3 | 17.4 |
| 100 rpm | 14.5 | 13.2 |
| 6 rpm | 6.1 | 5.3 |
| 3 rpm | 5.2 | 4.7 |
| Plastic viscosity, mPa.s (cP) | 8.6 | 7.0 |
| Yield point, Pa (lb/100 ft$^2$) | 6.75 (14.1) | 6.56 (13.7) |
| API Filtrate, ml | 11.4 | 12.0 |

These results show the excellent characteristics of an oil-well drilling fluid of the invention. In particular, the high viscosity at low shear rates provides good hold cleaning and the low viscosity at high shear rates increases the penetration rate of the bit.

Example 9

A French dressing is made using the following formulation:

| Ingredients | % | |
|---|---|---|
| Vegetable Oil | 38.00 | 38.00 |
| Water | 34.65 | 34.65 |
| Sugar | 11.50 | 11.50 |
| Vinegar (100 grain) | 9.00 | 9.00 |
| Salt | 4.00 | 4.00 |
| Paprika, powdered | 1.35 | 1.35 |
| Mustard, powdered | 1.25 | 1.25 |
| Low calcium, smooth flow xanthan gum | 0.25 | |
| Xanthan gum (Control) | | 0.25 |
| | 100.00% | 100.00% |

Procedure:

1. Dry blend xanthan gum with one-half of the sugar and hydrate with water and vinegar under vigorous agitation for 15 minutes.

2. Add blend of all remaining solids.

3. Add oil, slowly at first, then at normal rate.

4. Emulsify with a colloid mill at 0.02".

The flow properties of the dressings are measured in the Bostwick Consistometer using the procedure described in Example 3 with the following results:

Bostwick Results

| Seconds | 5 | 10 | Distance in cm at time indicated 20 | 30 | 60 | 120 | 180 | Visual examination of flow properties |
|---|---|---|---|---|---|---|---|---|
| Xanthan Gum | | | | | | | | |
| Low calcium | 10.0 | 10.8 | 11.7 | 12.5 | 13.6 | 14.7 | 15.3 | Smooth |
| Control | 5.0 | 5.8 | 6.6 | 7.2 | 8.1 | 9.0 | 9.5 | Chunky |

Example 10

A low calorie French dressing containing approximately 2.52J (0.6 calories) per millilitre or 12.6J (3 calories) per teaspoonful is made using the following formulation:

## 0 005 030

| Ingredients: | % | |
|---|---|---|
| Water | 55.95 | 55.95 |
| Vinegar (50 grain) | 18.00 | 18.00 |
| Tomato Paste (26%) | 7.50 | 7.50 |
| Vegetable Oil | 6.00 | 6.00 |
| Lemon Juice | 5.00 | 5.00 |
| Salt | 3.50 | 3.50 |
| Egg Yolk (fresh) | 2.00 | 2.00 |
| Paprika | 0.60 | 0.60 |
| Mustard | 0.50 | 0.50 |
| Low calcium, smooth flow xanthan gum | 0.75 | |
| Xanthan gum (Control) | | 0.75 |
| Onion powder | 0.10 | 0.10 |
| Garlic powder | 0.05 | 0.05 |
| Non-nutritive sweetener | 0.05 | 0.05 |
| | 100.00% | 100.00% |

Procedure:

1. Disperse the xanthan gum in the water and add with good agitation to all oil, vinegar and lemon juice in which the mustard is dispersed. Complete hydration in 10—15 minutes with stirring.

2. After hydration, add tomato paste and egg yolk.

3. Add blend of all solids with stirring.

4. Emulsify with a colloid mill at 0.015″ (0.038 cm).

The flow properties of the dressings are measured in the Bostwick Consistometer using the procedure described in Example 3 with the following results:

| | | | Distance in cm | | | | | Visual examination of |
|---|---|---|---|---|---|---|---|---|
| Seconds | 5 | 10 | 20 | 30 | 60 | 120 | 180 | flow properties |
| Xanthan Gum | | | | | | | | |
| Low calcium | 7.5 | 8.0 | 8.5 | 8.7 | 9.3 | 9.8 | 10.2 | Smooth |
| Control | 4.0 | 4.7 | 5.4 | 5.7 | 6.2 | 6.6 | 6.8 | Chunky |

Example 11

Various low calcium xanthan gum are produced according to the teachings of this invention and tested according to Test Method 1 and by visual observation with the following results:

| Sample | Visc. (cP) | Flow (visual determination) |
|---|---|---|
| 15 | 2350 | Very Chunky |
| 16a[1] | 1670 | Chunky |
| b | 1830 | Chunky |
| c | 1800 | Chunky |
| 17a | 1800 | Chunky |
| b | 1730 | Chunky |
| c | 1800 | Chunky |
| 18a | 1510 | Slightly Chunky |
| b | 1510 | Smooth |
| 19a | 1580 | Slightly Chunky |
| b | 1520 | Smooth |
| 20a | 1710 | Slightly Chunky |
| b | 1730 | Slightly Chunky |
| 21a | 1510 | Smooth |
| b | 1500 | Smooth |
| c | 1540 | Smooth |
| 22a | 1360 | Smooth |
| b | 1350 | Smooth |

[1] Letters indicate replicates of same sample, so that viscosity differences are within experimental error.

13

Example 12
Pilot plant fermentation
Smooth-flow, low calcium xanthan gum is prepared in a 4164-litre fermentor using soft water.

Inoculum: (378.5 litres)
    Age—54 1/2 hrs.
    pH—6.82
    viscosity—2550 cP
Medium: (4164 litres)

| | |
|---|---|
| Corn Syrup (D.S.) | 3.96% |
| $NH_4NO_3$ | 0.106% |
| $K_2HPO_4$ | 0.053% |
| Promosoy 100 | 0.033% |
| $MgSO_4 \cdot 7H_2O$ | 0.01% |
| Balab Defoamer | 0.25% (v/v) |
| K-60 Defoamer | 0.022 (v/v) |
| KOH | To control pH at 6.0—7.5 |

Fermentation:

| | |
|---|---|
| Beer pH | 7.06 |
| Temperature | 30—31°C |
| Aeration | 0.2—1.0 (v/v) |

Agitation:
    Disc and turbine impellors
    Number of sets: 3
    Number of blades/set: 5
    Disc diameter: 50.8 cm
    Blade dimension: 6.35 cm×10.16 cm
    Impellor diameter: 71.1 cm
    Speed—150 rpm
Recovery:
    Beer pH adjust to 6.0 with $H_2SO_4$
    Beer rate—315 ml.s (5 gpm)
    Pasteurization—74°C/6 min
    Ppt. with 3 volumes isopropanol

Following the procedure of Example 12 but replacing $K_2HPO_4$ with $Na_2HPO_4$, the smooth-flow, low calcium xanthan gum of the present invention is also produced.

**Claims**

1. A xanthan gum containing from 0.0037 to 0.04 weight per cent of calcium.

2. A xanthan gum as claimed in Claim 1 containing from 0.0037 to 0.02 weight % of calcium.

3. A smooth-flow xanthan gum as claimed in Claim 1.

4. A smooth-flow xanthan gum as claimed in Claim 2.

5. An aqueous composition containing a xanthan gum as claimed in Claim 3.

6. An aqueous composition as claimed in Claim 5 in the form of a water-in-oil emulsion.

7. A dry-mix composition comprising a foodstuff and an effective amount of low-calcium smooth-flow xanthan gum as claimed in Claim 3.

8. A toothpaste composition comprising glycerine, water, an abrasive, and an effective amount of a low-calcium smooth-flow xanthan gum as claimed in Claim 3.

**Revendications**

1. Une gomme de xanthane contenant de 0,0037 à 0,04% en poids de calcium.

2. Une gomme de xanthane comme revendiquée dans la revendication 1; contenant de 0,0037 à 0,02% en poids de calcium.

3. Une gomme de xanthane à écoulement régulier comme revendiquée dans la revendication 1.

4. Une gomme de xanthane à écoulement régulier comme revendiquée dans la revendication 2.

5. Une composition aqueuse contenant une gomme de xanthane comme revendiquée dans la revendication 3.

6. Une composition aqueous comme revendiquée dans la revendication 5 sous forme d'une émulsion eau dans l'huile.

7. Une composition mélangée sèche comprenant un aliment et une quantité efficace de gomme de xanthane à faible taux de calcium et à écoulement régulier comme revendiquée dans la revendication 3.

# 0 005 030

8. Une composition de pâte dentifrice comprenant de la glycénine, de l'eau et un abrasif et une quantité efficace d'une gomme de xanthane à faible taux de calcium et à écoulement régulier comme revendiquée dans la revendication 3.

## Patentansprüche

1. Xanthangummi, enthaltend 0,0037 bis 0,04 Gew.-% Calcium.
2. Xanthangummi nach Anspruch 1, enthaltend 0,0037 bis 0,02 Gew.-% Calcium.
3. Glatt-fliessender Xanthangummi nach Anspruch 1.
4. Glatt-fliessender Xanthangummi nach Anspruch 2.
5. Wässrige Zusammensetzung, enthaltend einen Xanthangummi nach Anspruch 3.
6. Wässrige Zusammensetzung nach Anspruch 5 in der Form einer Wasser-in-Öl-Emulsion.
7. Trockengemisch-Zusammensetzung, enthaltend ein Nahrungsmittel und eine wirksame Menge eines glatt-fliessenden Xanthangummis mit niedrigem Calciumgehalt gemäss Anspruch 3.
8. Zahnpastenzusammensetzung, enthaltend Glycerin, Wasser, ein Abriebmittel und eine wirksame Menge eines glatt-fliessenden Xanthangummis mit niedrigem Calciumgehalt nach Anspruch 3.